# EUROPEAN PATENT APPLICATION

(11) **EP 4 834 950 A1**
(43) Date of publication of application: **04.11.2026**
(21) Application number: 25173335.8
(22) Date of filing: 29.04.2025
(51) Int. Cl.: C08B 37/00, C08B 37/08, C08L 5/08, A61L 27/20

(54) **GLYCOSAMINOGLYCANS CONSISTING OF HYALURONIC ACID CROSS-LINKED WITH EITHER CHONDROITIN OR CHONDROITIN SULPHATE AND PROCESS FOR THEIR PREPARATION**

(71) Applicant: IBSA INSTITUT BIOCHIMIQUE SA, 6912 Lugano (CH)
(72) Inventor: DE ROSA, Mario, LUGANO (CH); RUGGIERO, Alessandro, LUGANO (CH); FOSSATI, Tiziano, LUGANO (CH); CARUCCI, Simone, 6912 LUGANO (CH)
(74) Representative: Bianchetti & Minoja with Trevisan & Cuonzo IPS SRL

(57) **Abstract**

Disclosed herein an innovative method to crosslink glycosaminoglycan (GAG) with a biphasic system method in which glycosaminoglycans remains in the solid state throughout the reticulation wherein the process according to the invention allows to obtain either hetero-crosslinked or homo-crosslinked forms of hyaluronic acid, chondroitin, chondroitin sulphate with optimized rheological properties for several clinical applications and in aesthetic medicine. Rheological characteristics of hetero-crosslinked or homo-crosslinked GAG can be modulated to appropriate homogenization treatments during the purification procedure.

## Description

The invention concerns glycosaminoglycans consisting of hyaluronic acid cross-linked with either chondroitin or chondroitin sulphate, hereinafter referred to as "hetero-cross-linked glycosaminoglycans (GAGs)". The invention also refers to process for the preparation of both hetero and homo-crosslinked glycosaminoglycans selected from hyaluronic acid, chondroitin or chondroitin sulphate comprising the reaction of a glycosaminoglycans or a mixture of two different glycosaminoglycan with a cross-linking agent in a two-phase system followed by homogenization during any step of the work-out phase.

### Background of the invention

Crosslinked glycosaminoglycans are used in anti-aging treatments and viscosupplementation thanks to their high biocompatibility, volumizing effect and low potential for adverse reactions. Dermal fillers have different viscoelastic characteristics depending, for example, on their degree of modification and especially cross-linking, concentration and particle size (Hyaluronic Acid Aesthetic Fillers: A review of Rheological and Physicochemical Properties 2023).

The viscoelasticity of dermal fillers is studied with rheology and allows to define the contribution of the viscous component and the elastic component of each filler. The definition of viscoelastic properties is necessary in order to choose the filler with appropriate characteristics for each patient and for the anatomical area in which it must be applied, since each area of the face is subjected to different mechanical forces that modify the shape, distribution, duration and level of correction that is desired. Some essential parameters to define the viscoelastic behavior of a glycosaminoglycans dermal filler are described below:
The viscous modulus (G"), expressed in Pascal (Pa), is the fraction of energy that dissipates after deformation, which confirms that the filler is not able to fully restore its initial shape after deformation, G" is clinically related to the injectability of the filler (higher G", means more difficult to extrude).

The elastic modulus (G'), expressed in Pascal (Pa), is the fraction of energy that the filler retains after deformation (in other words, its ability to resist deformation). Fillers with higher G' values are more suitable for deeper areas injection, because they are more structuring and volumizing, unlike those with lower G' values, which are softer and more suitable for more superficial areas. Usually, higher G' values make a gel more difficult to inject than lower values.

Tan δ corresponds to the G"/G' ratio and tells us whether a gel is more elastic or more viscous. If G' is higher and tan δ<1, the elastic component will be predominant; if G" is greater and tan δ>1, the viscous component will be predominant. Tan δ<1 is related to a gel behavior; the GAGs are not anymore in solution they are deformable solids. Keeping a low tan δ is therefore desired in order to preserve the elastic component of the system.

Another critical parameter that is taken into consideration for dermoaesthetic fillers is injectability, that refers to the force required to push a filler through a needle. The less force is required to extrude the filler from the needle, the easier and less painful the administration.

To produce fillers that remain for a certain period of time in the body, GAGs are stabilized with a cross-linking process that covalently links the polysaccharide chains together, giving rise to a non-soluble hydrogel slowly degraded in the body. The cross-linking process described in WO2013/164782 (De Rosa ep al.) is one of the most effective cross-linking processes, that involves the formation of cross-linked homo-polymers of GAGs. "Cross-linked homo-polymer" here means chains of the same polymer cross-linked to each other whereas "cross-linked hetero-polymer refers to a cross-linked glycosaminoglycan wherein two different glycosaminoglycans are cross linked through chemical bonds between the functional group present on one species with functional groups present on the other species. Examples of hetero-polymers may be obtained for instance by cross-linked hyaluronic acid to chondroitin or hyaluronic acid to chondroitin sulfate, optionally in salified form. Said heteropolymers are new and are a first object of the invention, The process according to WO2013/164782 differs from crosslinking in solution essentially in a in the reaction carried out in two-phase system (defined as a non-solvent system). This type of approach guarantees a final product that maintains a limited degradation of the molecular weight of the initial GAG chain compared to known processes. In the latter, in fact, the reaction conditions are such as to favor both the formation of the actual crosslinked product and the hydrolysis of the glycosidic bonds present in the initial GAG chain, with a consequent decrease in its molecular weight. This last reaction, according to the process disclosed in WO2013/164782 is limited. The characteristic of the final product obtained is the maintenance of the three-dimensional characteristics of the starting glycosaminoglycan (shape memory). Furthermore, WO2013/16478, by limiting the reaction volumes, reaction times and purification procedure, involves reduced costs and is therefore more convenient.

WO2013/164782 teaches that the reaction product consists of a continuous gel that can undergo mechanical treatments to reduce its particle size between 50-500 µm. WO2013/164782 teaches that such treatments can be inserted downstream of the process, after the final recovery of the cross-linked product has occurred. This product is subsequently hydrated in buffered solution in order to obtain the desired final concentration and sterilized.

Examples of mechanical treatments used to obtain the desired particle size are for example sieving and grinding.

EP 3 710 526 discloses implantable devices based on crosslinked glycosaminoglycans obtained by a process comprising the formation of hybrid complexes of a low molecular weight glycosaminoglycan and an high molecular weight hyaluronic acid followed by crosslinking said complexes with a diepoxide. Crosslinked complexes of low and high molecular weight hyaluronic acids have been specifically disclosed.

### Description of the invention

In a first aspect, the invention provides novel glycosaminoglycans consisting of hyaluronic acid cross-linked with either chondroitin or chondroitin sulphate (hetero-crosslinked GAG polymer). Cross-linking between the functional groups present on the hyaluronic acid chain on one side and the functional groups present on the chondroitin or chondroitin sulfate chains on the other side is preferably obtained through a polyfunctional epoxy crosslinking agent, preferably 1,4-butanediol diglycidyl ether.

The cross-linked hetero- glycosaminoglycans of the invention are characterized by biological activities typical of glycosaminoglycans and show positive effects on stability at the implant sites and on synergies in the elicited biological activities.

In a second aspect, the invention provides a new process which differs substantially from the process of WO2013/164782, allowing to obtain both the crosslinked hetero-polymers of the invention and cross-linked hyaluronic acid, cross-linked chondroitin or cross-linked chondroitin sulphate (homo- cross-linked polymers) with optimized rheological and extrudability properties, thanks to a critical homogenization treatment, allowing to simultaneously fine-tuning the tan δ and the extrusion force. The homogenization step, differently from the process disclosed in WO2013/164782, must performed in any step of the purification process after the crosslinking reaction but before the final recovery of the purified crosslinked product. The process of the invention allows to obtain the simultaneous reduction of both tan δ and extrusion force compared to the values obtained by conducting homogenization as the final step after the recovery step of the cross-linked product. This step can be performed at any point in the process and for any duration, without impacting overall process efficiency.

### Detailed description of the invention

It has been found that glycosaminoglycans of different species, e.g. hyaluronic acid, chondroitin and chondroitin sulfates, can be advantageously cross-linked together providing a novel class of cross-linked glycosaminoglycan which can be successfully used for medical, pharmaceutical or cosmetic applications. Examples of the cross-linked hetero GAGs of the invention include hyaluronic acid (or a salt thereof) cross-linked with chondroitin or hyaluronic acid (or a salt thereof) cross-linked with chondroitin sulfate. Cross-linking may be effected by means of polyfunctional epoxy crosslinking agents or equivalent reagents providing an ester-type or ether type covalent bond between the hydroxy and carboxy groups of the glycosaminoglycans.

For the purposes of the invention, the molecular weight of the glycosaminoglycan to be cross-linked may be selected in a wide interval, depending on the considered use. For instance, in a range from about 10KDa to about 3MDa evaluated by a Size Exclusion Chromatography (SEC-TDA triple detector array).

The process for the preparation of crosslinked hetero or homo-glycosaminoglycans of hyaluronic acid, chondroitin and chondroitin sulphate and their salts, which comprises: cross-linking a glycosaminoglycan or a mixture of at least two different glycosaminoglycans in a non-solvent system consisting of mixtures of organic solvents and water in the presence of a quaternary ammonium base and of a polyfunctional epoxy crosslinking agent, followed by purification, characterized in that at least one homogenization step is performed during the purification process. The non-solvent system consists of mixtures of organic solvents and water with a volume ratio between organic solvent and water > 2.

Examples of suitable organic solvents are ketones, alcohols and ethers miscible with water. Acetone is particularly preferred.

Examples of quaternary ammonium bases are tetrabutyl ammonium hydroxide, tetrapropyl ammonium hydroxide, tetraethyl ammonium hydroxide, tetramethyl ammonium hydroxide, benzyl tributyl ammonium hydroxide, benzyl tripropyl ammonium hydroxide, benzyl triethyl ammonium hydroxide, benzyl trimethyl ammonium hydroxide, methyl tributyl ammonium hydroxide, methyl tripropyl ammonium hydroxide, methyl triethyl ammonium hydroxide, phenyl tributyl ammonium hydroxide, phenyl tripropyl ammonium hydroxide, phenyl triethyl ammonium hydroxide, phenyl trimethyl ammonium hydroxide, dodecyl trimethyl ammonium hydroxide, tetradecyl trimethyl ammonium hydroxide, hexadecyl trimethyl ammonium hydroxide, octadecyl trimethyl ammonium hydroxide and choline hydroxide. Said bases are preferably used at concentrations ranging between 0.125 and 1.3 M. Benzyl trimethyl ammonium hydroxide is particularly preferred. The polyfunctional epoxy crosslinking agent is selected from 1,3-butadiene diepoxide, 1,2,7,8-diepoxyoctane, epoxy 1,5-hexadiene, ethylene glycol diglycidyl ether, 1,4-butanediol diglycidyl ether, 1,6-hexanediol diglycidyl ether, polyethylene glycol diglycidyl ether, polypropylene glycol diglycidyl ether and bisphenol A diglycidyl ether. 1,4-butanediol diglycidyl ether, polyethylene glycol and polypropylene glycol diglycidyl ether are preferred.

The reaction can be conducted at temperatures between 20 and 80°C, for reaction times between 0.5 and 10 h, preferably between 2 and 16 h.

The process is preferably carried out using mixtures of organic solvents and water in the presence of benzyl trimethyl ammonium hydroxide in concentrations from 0.025 to 1.3 M and 1,4-butanediol diglycidyl ether in a molar ratio < 20% of 1,4-butanediol diglycidyl ether/glycosaminoglycan OH groups, at a temperature from 20 to 80°C, for reaction times from 0,5 to 10 h.

According to a first alternative (Examples 2A-2C), the purification comprises the steps of:
a) removing the liquid phase;
b) washing of the solid fraction;
c) hydration and homogenization;
d) intermediate recovery by precipitation with ethanol;
e) hydration, precipitation and removal of the liquid phase;
f) final recovery by dehydration and drying.

According to a second alternative (Example 3), the purification process comprises the steps of:
a) removing the liquid phase;
b) washing of the solid fraction;
c) hydration, precipitation and removal of the liquid phase;
d) hydration and homogenization;
e) final recovery by dehydration and drying.

According to a further alternative (example 4), the purification process comprises the steps of:
a) removing the liquid phase;
b) washing of the solid fraction;
c) hydration and homogenization;
d) intermediate recovery by precipitation with ethanol;
e) hydration and homogenization;
f) final recovery by dehydration and drying.

When the reaction is complete, it can be stopped by adding an acid such as H₃PO₄.

The reaction mixture is then filtered, removing the liquid phase containing the reagents and by products. The separated solid is then washed with acetone or other solvent. The intermediate recovery, after or before the homogenization step, is typically performed by adding ethanol in which the cross-linked polymer is insoluble. The homogenization step comprises hydration of the solid fraction obtained by the first washing step and/or of the solid fraction obtained by the intermediate recovery by precipitation with ethanol followed by colloidal milling, sieving, filtration by extrusion or their combinations. The duration of the homogenization is not critical and may vary between wide ranges, for instance from 2 to 15 minutes or more.

The final purification steps include a hydration step with a saline solution, followed by precipitation with ethanol and removal of the liquid phase. This step may be repeated twice. Dehydration with acetone, filtration and drying according to conventional methods yield the desired cross-linked homo-or hetero-polymers.

The invention, in a further aspect, also provides compositions for medical, cosmetical uses comprising the cross-linked hetero-polymers or the cross-linked homo-polymers obtained according to the disclosed process.

The examples below describe the invention in more detail.

### Comparative Example 1A - Synthesis of HA crosslinked homopolymer according to WO2013/164782

In WO2013/164782 the reaction is only conducted with a single GAG: hyaluronic acid or its salts (HA) or biotechnological chondroitin and its salts (CB) or extractive chondroitin sulfate and its salts (CS), not with mixes of different GAGs, to give crosslinked homo-polymers, in this example HA crosslinked homo-polymer.
1. Preparation of reaction mix - 200 g of HA powder and 1,4-butanediol diglycidyl ether (BDDE) (weight ratio BDDE/HA = 6%) were added to 250 mmols of benzyl trimethyl ammonium hydroxide (TMBAH) (2.5 M aqueous solution) in 1264 mL of acetone and 356 mL of water.
2. Reaction - The reaction mixture was stirred mechanically at 100-120 rpm, the reaction temperature was brought to 61°C, reaction time 3 h±0.5 h
3. Reaction stop - The reaction was stopped by addition of 16 mL H₃PO₄ in 984 mL of acetone to pH 7.4.
4. Removal of the liquid phase - Reagents and reaction by-products are present in the liquid phase removed by filtration.
5. First purification process - 5 successive washes with 3 L of acetone.
6. Hydration/precipitation/removal of liquid phase -homo-crosslinked was hydrated with 13 L of aqueous NaCl solution (0.9% w/v) and precipitated with 40 L of ethanol 99.9%. The removal of liquid phase completed the process. This operation sequence was repeated twice.
7. Final recovery of the crosslinked product - The precipitated homo-crosslinked HA was dehydrated by treating with 10 L of acetone, removal of the supernatant with a vacuum filtration system (Buchner funnel), this process was repeated 2 times. Drying first at 2-7°C for 16-20 h and then at 60-70°C for 16-20 h completed the drying process.

The final product was a powder insoluble in both acetone and ethanol.

The crosslinked product has been characterized for viscoelastic properties and extrudability forces as follows.

### Characterization of Crosslinked product

The powder of crosslinked product has been analysed at a final concentration of 25mg/ml. The powder has been dissolved in PBS buffer, made up of sodium phosphate dibasic anhydrous 0.055 %, sodium phosphate monobasic dehydrate 0.009%, distilled water and NaCl at a concentration to guarantee a final Osmolality of 300 ± 50 mOsm.

A flacon with PBS is put in a thermostated thermoshaker at 80°C. Once the temperature of 80°C has been reached, the crosslinked powder, previously weighed, has been added and vortexed for 1 minute in order to avoid the formation of lumps. At the end of the operation, the flacon has been put into the thermoshaker and shaked at 1000rpm for 3 hours maintaining the temperature at 80°C. At the end, the sample has been cooled down by setting a temperature of 25°C. The sample has been left to rest for 21 hours at room temperature. Finally, the product has been divided into 1 mL long syringes (overage 0.1 mL) and has been sterilized (121°C for 15 minutes).

The viscoelastic properties of the product obtained have been evaluated applying the "Frequency Sweep Test" using the MCR 702e MultiDrive rheometer (Anton Paar, Germany) equipped with a CP-50 2 geometry (cone diameter 49.970 mm, cone angle 1.995°, truncation 207 µm) and a Peltier temperature control. The "Frequency Sweep Test" has been conducted by varying the frequency of the applied stress, from 0.159 to 15Hz at 37°C, at a constant strain selected within the linear viscoelasticity range (0.1%). The values of G', G", expressed in Pascal (Pa), and tanδ are extrapolated at 1Hz from three measurements of each sample and reported as mean ± standard deviation.

The extrusion force of the product has been evaluated using the Texture analyser TH-HD PLUS applying the compression test at a speed of 12mm / min. Data is acquired every 200 point*sec and the extrusion force, expressed in Newtons (N), is obtained by averaging all points in the plateau region between 25 and 50 seconds. The results of viscoelasticity properties and extrusion force of the product obtained in this example without homogenization were G' 1132 Pa; G" 124.52 Pa; tan δ G"/G' 0.11; Extrusion force (Injectability) 18 N.

### Reference Example 1B - Synthesis of HA/CB crosslinked heteropolymer according to comparative Example 1A

The reaction was conducted with a combination of 140 g HA and 60 g CB.

The final product was a powder insoluble in both acetone and ethanol.

The crosslinked powder has been characterized following the methods reported in Example 1A.

The results of viscoelasticity properties and extrusion force of the HA/CB crosslinked product obtained without homogenization treatment were: G' 451 Pa; G" 54.12 Pa; tan δ G"/G' 0.12; Extrusion force (Injectability) 14 N.

### Example 2A - Synthesis of HA/CB crosslinked hetero-polymer with homogenization treatment after initial purification

Preparation of reaction mix - 140 g of HA + 60 g of CB powders and 12 mL of BDDE (weight ratio BDDE/HA = 6%) were added to 200mL of TMBAH (2.5 M aqueous solution) in 1264 mL of acetone and 356 mL of water.
2. Reaction - The reaction mixture was stirred at 100-120 rpm and the temperature brought to 61°C for 3 h±0.5 h
3. Stop of the reaction - The reaction was stopped by addition of 16 mL of H₃PO₄ in 984 mL of acetone to pH 7.4.
4. Removal of the liquid phase - The reagents and reaction by-products were present in the liquid phase removed by filtration.
5. First purification process - The solid was washed with 5 X 3 L of acetone.
6. Homogenization (grinding in colloidal mill) -The hetero-crosslinked HA/CB was hydrated with 13 L of 0.9% w/v NaCl solution. The swollen hetero-crosslinked HA/CB was ground in colloidal mill for 5 min.
7. Intermediate recovery - The recovery of the crosslinked product was performed adding 40 L of ethanol 99.9%. The final product was insoluble in ethanol.
8. Hydration/precipitation/removal of liquid phase - The hetero-crosslinked HA/CB was hydrated with 13 L of aqueous NaCl solution (0.9% w/v) and the hydrated product was precipitated with 40 L of ethanol 99.9%. The removal of liquid phase completed the process. This operation sequence was repeated twice.
9. Final recovery of the crosslinked product - Dehydration of precipitated hetero-crosslinked HA/CB was obtained by treating twice with 10 L of acetone and removing the supernatant with a vacuum filtration system (Buchner funnel). Drying first at 2-7°C for 16-20 h and then at 60-70°C for 16-20 h completed the drying process.

The final product was a powder insoluble in both acetone and ethanol.

The crosslinked powder has been characterized following the methods reported in Example 1A.

The results of viscoelasticity properties and extrusion force of the HA/CB crosslinked product obtained with homogenization treatment (point 6) for 5 minutes, were: G' 257.21 Pa; G" 21.77 Pa; tan δ G"/G' 0.08; Extrusion force 6N (Injectability).

The results show that homogenization after the first purification steps improves the tan δ value and the extrusion force compared to the results obtained in Example 1B.

### Example 2B - Synthesis of HA/CB crosslinked hetero-polymer with homogenization treatment in colloidal mill

1. Preparation of reaction mix - 140 g of HA + 60 g of CB powders and 12 mL of BDDE (weight ratio BDDE/HA-CB = 6%) were added to 200mL of TMBAH (2.5 M aqueous solution) in 1264 mL of acetone and 356 mL of water.
2. Reaction - The reaction mixture was stirred mechanically at 100-120 rpm, the temperature was brought to 61°C, reaction time 3 h±0.5 h
3. Stop of the reaction - The reaction was stopped by adding 16 mL of H₃PO₄ in 984 mL of acetone up to pH 7.4.
4. Removal of the liquid phase- The reagents and reaction by-products were present in the liquid phase removed by filtration.
5. First purification process - The solid was washed with 5 X 3 L of acetone.
6.Homogenization (grinding in colloidal mill) - Hydration of the hetero-crosslinked HA/CB with 13 L of 0.9% w/v NaCl solution. The swollen hetero-crosslinked HA/CB was ground in colloidal mill for 10 min.
7. Intermediate recovery - The recovery of the crosslinked product was performed by adding 40 L of ethanol 99.9%. The final product was insoluble in ethanol.
8. Hydration/precipitation/removal of liquid phase - The hetero-crosslinked HA/CB was hydrated with 13 L of aqueous NaCl solution (0.9% w/v) and precipitated with 40 L of ethanol 99.9%. The removal of liquid phase completed the process. This operation sequence was repeated twice.
9. Final recovery of the crosslinked product - The precipitated hetero-crosslinked HA/CB was dehydrated by treating twice with 10 L of acetone and removal of the supernatant with a vacuum filtration system (Buchner funnel). Drying first at 2-7°C for 16-20 h and then at 60-70°C for 16-20 h completed the drying process.

The final product was a powder insoluble in both acetone and ethanol.

The crosslinked powder has been characterized following the methods reported in Example 1A.

The results of viscoelasticity properties and extrusion force of the HA/CB crosslinked product obtained with the homogenization treatment (point 6) for 10 minutes, were: G' 167.94 Pa; G" 14.74 Pa; tan δ G"/G' 0.09; Extrusion force 5N (Injectability).

The results show that the homogenization treatment is duration-independent and improves the tan δ value and the extrusion force compared to the results obtained in Example 1B.

### Example 2C - Synthesis of HA/CB crosslinked hetero-polymer with homogenization by grinding

1.Preparation of reaction mix - 140 g of HA + 60 g of CB powders and 12 mL of BDDE (molar ratio BDDE/HA-CB OH groups = 6%) were added to 200 mL of TMBAH (2.5 M aqueous solution) in 1264 mL of acetone and 356 mL of water.
2. Reaction - The reaction mixture was stirred mechanically at 100-120 rpm, the temperature was brought to 61°C, reaction time 3 h±0.5 h
3. Stop of the reaction - The reaction was stopped by adding 16 mL of H₃PO₄ in 984 mL of acetone up to pH 7.4.
4. Removal of the liquid phase - The reagents and reaction by-products were present in the liquid phase removed by filtration.
5. First purification process - The solid was washed with 5 X 3 L of acetone.
6. Homogenization (grinding in colloidal mill) -The hetero-crosslinked HA/CB was hydrated with 13 L of 0.9% w/v NaCl solution. The swollen hetero-crosslinked HA/CB was ground in colloidal mill for 20 min.
7. Intermediate recovery -The crosslinked product was recovered by adding 40 L of ethanol 99.9%. The final product was insoluble in ethanol.
8. Hydration/precipitation/removal of liquid phase - The hetero-crosslinked HA/CB was hydrated with 13 L of aqueous NaCl solution (0.9% w/v) and precipitated with 40 L of ethanol 99.9%. The removal of liquid phase completed the process. This operation sequence was repeated twice.
9. Final recovery of the crosslinked product -The precipitated hetero-crosslinked HA/CB was dehydrated by treating twice with 10 L of acetone, the supernatant was removed with a vacuum filtration system (Buchner funnel). Drying first at 2-7°C for 16-20 h and then at 60-70°C for 16-20 h completed the drying process.

The final product was a powder insoluble in both acetone and ethanol.

The crosslinked powder has been characterized following the methods reported in Example 1A.

The results of viscoelasticity properties and extrusion force of the HA/CB crosslinked product obtained with homogenization treatment (point 6) for 20 minutes, were: G' 174.63 Pa; G" 15.92 Pa; tan δ G"/G' 0.09; Extrusion force 5N (Injectability).

The results confirm that the duration of homogenization treatment is not critical and does not affect the overall process efficiency, improving the tan δ value and the extrusion force compared to the results obtained in Example 1B.

### Example 3 - Synthesis of HA/CB crosslinked heteropolymer with homogenization Steps 1-5 were repeated as reported in example 2A.

6. Hydration/precipitation/removal of liquid phase -The hetero-crosslinked HA/CB was hydrated with 13 L of aqueous NaCl solution (0.9% w/v) and precipitated with 40 L of ethanol 99.9%. The removal of liquid phase completed the process. This operation sequence was repeated twice.
7. The hetero-crosslinked HA/CB was hydrated with 13 L of 0.9% w/v NaCl solution.
8. Homogenization (grinding in colloidal mill) - The swollen hetero-crosslinked HA/CB was ground in colloidal mill for 20 min.
9. Final recovery of the hetero-crosslinked HA/CB - Precipitation in ethanol first and then acetone, followed by removal of the supernatant and subsequent recovery of the powder with a vacuum filtration system (Buchner funnel). The product was then dried at 2-7°C for 16-20 h and then 60-70°C for 16-20 h. The final product was insoluble in both acetone and ethanol.

The crosslinked powder has been characterized following the methods reported in Example 1A.

The results of viscoelasticity properties and extrusion force of the HA/CB crosslinked product obtained with homogenization treatment (point8) for 20 minutes, were: G' 174.45 Pa; G" 15.51 Pa; tan δ G"/G' 0.09; Extrusion force 6N (Injectability).

The results show that homogenization can be performed at any point in the process, without impacting overall process efficiency, improving the tan δ value and the extrusion force compared to the results obtained in Example 1B.

### Example 4 - Synthesis of HA/CB crosslinked heteropolymer with grinding

Steps 1-7 as reported in example 2B.
8. Homogenization (2nd grinding in colloidal mill) - A solution of NaCl was added to the product with subsequent grinding step (10 min).
9. Final recovery of the hetero-crosslinked HA/CB - Precipitation in ethanol first and then acetone, followed by removal of the supernatant and subsequent recovery of the powder with a vacuum filtration system (Buchner funnel). Subsequent drying at 2-7°C for 16-20 h and then 60-70°C for 16-20 h. The final product was insoluble in both acetone and ethanol.

The crosslinked powder has been characterized following the procedures of characterization reported in Example 1A.

The results of viscoelasticity properties and extrusion force of the HA/CB crosslinked product obtained with homogenization treatment (points 6 and 8) for 10 minutes each, were: G' 231.92 Pa; G" 20.17 Pa; tan δ G"/G' 0.09; Extrusion force 4N (Injectability).

The results confirm that the homogenization treatment can be performed at any point in the process, without impacting overall process efficiency, improving the tan δ value and the extrusion force compared to the results obtained in Example 1B.

### Example 5 - Synthesis of HA/HA crosslinked homopolymer with homogenization The process is the same as reported in example 3 but using only HA (200 g)

The final product is insoluble in both acetone and ethanol.

The crosslinked powder has been characterized following the methods reported in Example 1A.

The results of viscoelasticity properties and extrusion force of the HA/HA crosslinked product obtained with homogenization treatment (point 8) for 20 minutes, were: G' 783.62 Pa; G" 66.03 Pa; tan δ G"/G' 0.08; Extrusion force 9N (Injectability).

The results show that the homogenization treatment can be performed at any point in the process, without impacting overall process efficiency, improving the tan δ value and the extrusion force improve compared to the results obtained in Example 1A.

### Example 6 - Synthesis of HA/HA crosslinked homopolymer with homogenization The process is the same as reported in example 4 but using only HA (200 g)

The final product was insoluble in both acetone and ethanol.

The crosslinked powder has been characterized following the methods reported in Example 1A.

The results of viscoelasticity properties and extrusion force of the HA/HA crosslinked product obtained with homogenization treatment (point 6 and 8) for 10 minutes each t, were: G' 811.77 Pa; G" 71.31 Pa; tan δ G"/G' 0.09; Extrusion force 10N (Injectability).

The results confirm that the homogenization can be performed at any point in the process and for any duration, without affecting the overall process efficiency, improving the tan δ value and the extrusion force improve compared to the results obtained in the Example 1A.

## Claims

1. Glycosaminoglycans consisting of hyaluronic acid cross-linked with either chondroitin or chondroitin sulphate.

2. Glycosaminoglycans according to claim 1 wherein hyaluronic acid is cross-linked to chondroitin or chondroitin sulfate by means of a polyfunctional epoxy crosslinking agent, preferably 1,4-butanediol diglycidyl ether.

3. Glycosaminoglycans according to claim 1 obtained by cross-linking hyaluronic acid with either chondroitin or chondroitin sulphate in a non-solvent system consisting of mixtures of organic solvents and water in the presence of benzyl trimethyl ammonium hydroxide in concentrations from 0.025 to 1.3 M and 1,4-butanediol diglycidyl ether in a molar ratio < 20% of 1,4-butanediol diglycidyl ether/glycosaminoglycan OH groups, at a temperature from 20 to 80°C, for reaction times from 0,5 to 10 h.

4. A process for the preparation of crosslinked hetero or homo-glycosaminoglycans of hyaluronic acid, chondroitin and chondroitin sulphate and their salts, which comprises cross-linking a glycosaminoglycan or a mixture thereof with a different glycosaminoglycan in a non-solvent system consisting of mixtures of organic solvents and water in the presence of a quaternary ammonium base and of a polyfunctional epoxy crosslinking agent, followed by purification,
**characterized in that** at least one homogenization step is performed during the purification process.

5. A process according to claim 4 wherein the purification comprises the steps of:
a) removing the liquid phase;
b) washing of the solid fraction;
c) hydration and homogenization;
d) intermediate recovery by precipitation with ethanol;
e) hydration, precipitation and removal of the liquid phase;
f) final recovery by dehydration and drying.

6. A process according to claim 4 wherein the purification comprises the steps of:
a) removing the liquid phase;
b) washing of the solid fraction;
c) hydration, precipitation and removal of the liquid phase;
d) hydration and homogenization;
e) final recovery by dehydration and drying.

7. A process according to claim 4 wherein the purification comprises the steps of:
a) removing the liquid phase;
b) washing of the solid fraction;
c) hydration and homogenization;
d) intermediate recovery by precipitation with ethanol;
e) hydration and homogenization;
f) final recovery by dehydration and drying.

8. A process according to claims 4-7 wherein the homogenization step comprises hydration of the solid fraction obtained by the first washing step and/or of the solid fraction obtained by the intermediate recovery by precipitation with ethanol followed by colloidal milling, sieving, filtration by extrusion or their combinations.

9. A process according to any one of claims 4-8 wherein the non-solvent system consists of mixtures of organic solvents and water with a volume ratio between organic solvent and water > 2.

10. A process according to claims 4-9 wherein the organic solvents are selected from ketones, alcohols and ethers miscible with water, preferably acetone.

11. A process according to any one of claims 4-10 wherein the quaternary ammonium base is selected from tetrabutyl ammonium hydroxide, tetrapropyl ammonium hydroxide, tetraethyl ammonium hydroxide, tetramethyl ammonium hydroxide, benzyl tributyl ammonium hydroxide, benzyl tripropyl ammonium hydroxide, benzyl triethyl ammonium hydroxide, benzyl trimethyl ammonium hydroxide, methyl tributyl ammonium hydroxide, methyl tripropyl ammonium hydroxide, methyl triethyl ammonium hydroxide, phenyl tributyl ammonium hydroxide, phenyl tripropyl ammonium hydroxide, phenyl triethyl ammonium hydroxide, phenyl trimethyl ammonium hydroxide, dodecyl trimethyl ammonium hydroxide, tetradecyl trimethyl ammonium hydroxide, hexadecyl trimethyl ammonium hydroxide, octadecyl trimethyl ammonium hydroxide and choline hydroxide, preferably benzyl trimethyl ammonium hydroxide.

12. A process according to any one of claims 4- 11 wherein the polyfunctional epoxy crosslinking agent is selected from 1,3-butadiene diepoxide, 1,2,7,8-diepoxyoctane, epoxy 1,5-hexadiene, ethylene glycol diglycidyl ether, 1,4-butanediol diglycidyl ether, 1,6-hexanediol diglycidyl ether, polyethylene glycol diglycidyl ether, polypropylene glycol diglycidyl ether and bisphenol A diglycidyl ether, preferably 1,4-butanediol diglycidyl ether.

13. A process according to any one of claims 4-12 wherein the reaction is performed in the presence of benzyl trimethyl ammonium hydroxide in concentrations from 0.025 to 1.3 M and 1,4-butanediol diglycidyl ether in a weight ratio < 20% of 1,4-butanediol diglycidyl ether/glycosaminoglycans, at temperatures from 20 to 80°C, for reaction times from 0, 5 to 10 h.

14. Crosslinked homo glycosaminoglycans obtained by the process of claims 4 -13.

15. A composition comprising the crosslinked hetero- glycosaminoglycans of claims 1-4 or the crosslinked homo glycosaminoglycans of claim 14.
